(19) Europäisches Patentamt
European Patent Office
Office européen des brevets

(11) Publication number: **0 219 352 A2**

(12) **EUROPEAN PATENT APPLICATION**

(21) Application number: 86307989.3

(22) Date of filing: 15.10.86

(51) Int. Cl.[4]: C 12 Q 1/28
G 01 N 33/52, G 01 N 33/58,
G 01 N 33/533

(30) Priority: 15.10.85 US 787525

(43) Date of publication of application:
22.04.87 Bulletin 87/17

(84) Designated Contracting States:
BE CH DE FR GB IT LI NL SE

(71) Applicant: **MINNESOTA MINING AND MANUFACTURING COMPANY**
3M Center, P.O. Box 33427
St. Paul, Minnesota 55133-3427 (US)

(72) Inventor: **Milbrath, Dean S. Minnesota Mining Man. Comp.**
2501 Hudson Road P.O. Box 33427
St. Paul Minnesota 55133-3427 (US)

(74) Representative: **Baillie, Iain Cameron et al**
c/o Ladas & Parry Isartorplatz 5
D-8000 München 2 (DE)

(54) **Chemiluminescent methods and kit.**

(57) Amines act as catalysts for enhancing the light output of chemiluminescent reactions based on amino-substituted cyclic diacylhydrazides such as the luminol reaction, resulting in increased sensitivity and utility of the reactions in diagnostic assays.

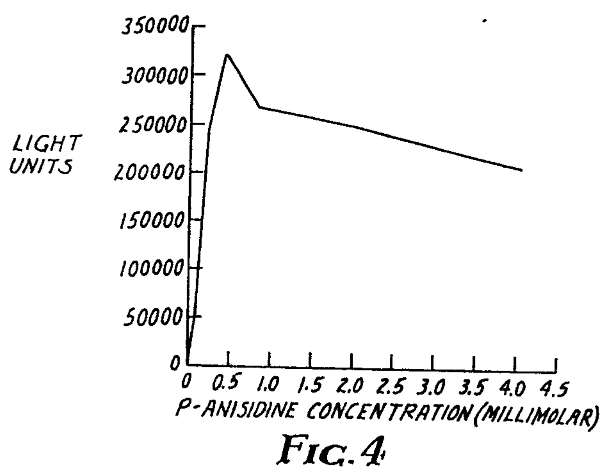


FIG. 4

EP 0 219 352 A2

## Description

# CHEMILUMINESCENT METHODS AND KIT

## TECHNICAL FIELD

The present invention relates to a method for enhancing the light output of a chemiluminescent reaction, and to a method for detecting components of chemiluminescent reactions. The present invention also relates to an assay kit for conducting certain of these assays.

## BACKGROUND OF THE INVENTION

A few chemical compounds exhibit chemiluminescent behavior, and they are generally valued for this property. Luminol (5-amino-2,3-dihydro-1,4-phthalazinedione) is well known for its ability to emit light when oxidized. It is used in a number of analytical methods, for example, to analyze for trace metal ions, copper, iron, peroxides and cyanides.

The light emission of luminol is typically elicited by combining it with an oxidizing agent such as hydrogen peroxide in the presence of a catalyst for an oxidation reaction such as a peroxidase. This reaction is called the "luminol reaction" herein. It has been reported by Whitehead, et al., Nature, 305, 158-159 (1983) that the addition of firefly luciferin to a luminol reaction causes enhanced light output.

Assays using chemically induced fluorescence are known to the art. For example, see U. S. Patent No. 4,220,450.

U. S. Patent No. 4,433,060 discloses a chemiluminescent immunoassay utilizing a triphenylmethane dye activated by a peroxide and chloramine. The triphenylmethane dye is used as a labelling substance.

Yurow et al., Analytica Chimica Acta., 68, pp. 203-204 (1974), discloses an assay for certain ketones employing a chemiluminescence reaction involving luminol.

A method for the enhancement of light output from chemiluminescent reactions has previously been described by the inventor in U. S. Serial No. 597,483. The method of that application utilizes beta-lactam antibiotics as co-reactants.

Another method for the enhancement of light output from chemiluminescent reactions such as the luminol reaction has been described by G. H. G. Thorpe et al. in J. Immunological Methods, 79, 57-63 (1985) and in European Patent Application No. 84300725.3. This method requires the use of aromatic acids (phenols) as catalysts.

The Derwent abstract for U.S.S.R. Patent 76SU-360442 describes chemiluminescent analysis of potato tubers infected with phytopathogenic microorganisms wherein potato juice diluted with water is treated with luminol, hydrogen peroxide and benzidine and the analytical time required for the chemiluminescence test is reduced to 34 minutes.

Luminescence has been employed in at least three major luminescent or luminometric immunoassay systems:

(a) Organoluminescent or organoluminometric immunoassays wherein chemiluminescent or bioluminescent compounds which participate directly in luminescent reactions (i.e., which are converted to an excited state and then returned to a non-excited state with the emission of a photon) have been used to label ligands such as proteins, hormones, haptens, steroids, nucleic acids, metabolites, antigens and/or antibodies. Examples of suitable compounds include luminol and isoluminol;

(b) Luminescent catalyst or cofactor immunoassays wherein catalysts or cofactors of luminescent reactions have been used as labels. An example of a suitable catalyst is the enzyme peroxidase; and

(c) Enzyme-linked immunoassays wherein luminescent reactions have been used to determine the products formed by the action of enzyme labels on suitable substrates. An example of this type of immunoassay is the determination of antibody linked glucose oxidase by the reaction of the enzyme/antibody reagent with glucose to form hydrogen peroxide and then measuring the amount of hydrogen peroxide produced by adding luminol under controlled conditions to initiate a luminescent reaction.

The sensitivity of the above immunoassays is determined in part by the lower limit for detection of the label or the product of the label. In the case of luminescent or luminometric immunoassays, the sensitivity of the system will depend partially on the light emitted in the luminescent reaction per unit of labelled material.

Examples of assays which are not immunoassays but which may incorporate luminescent reactions include:

(a) An elastase assay based on the release of peroxidase from an insoluble peroxidase-elastin preparation,

(b) A glucose assay based on co-immobilized glucose oxidase and peroxidase, and

(c) An assay of a peroxidase enzyme, an amino-substituted cyclic diacylhydrazide, or an oxidant, such as hydrogen peroxide, when these materials are neither labels nor the products of labels.

## SUMMARY OF THE INVENTION

The present invention relates to the use of certain amines to enhance the chemiluminescence of reactions such as the luminol reaction. More specifically, the chemiluminescent behavior of such amines in conjunction with luminol and other chemiluminescent compounds is useful in the luminescent detection of specific

components of the chemiluminescent reaction as a result of increasing the sensitivity of the reaction by using the amine compound. Finally, the present invention also relates to an assay kit for conducting chemiluminescent assays.

More particularly, one method of the invention is for enhancing the sensitivity of an assay reaction for the detection of a component of a chemiluminescent reaction involving a chemiluminescent compound, an inorganic peroxide source, and an oxidation catalyst. The component being assayed for may be any of the foregoing. This method comprises including an amine in the assay reaction in order to increase the sensitivity thereof. The amine used is selected from the group consisting of:

(a) a mono-cyclic aniline compound of the formula:

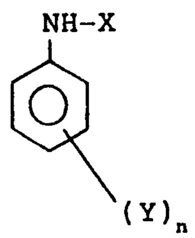

wherein X is hydrogen, methyl, or ethyl; each Y independently is an electron-donating group; and n is zero, one, two or three;

(b) a biphenyl compound of the formula:

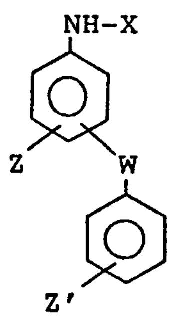

wherein X is hydrogen, methyl or ethyl; W is a carbon-carbon bond, oxygen, methylene, or $-CH_2O-$; and Z and Z′ are independently hydrogen, halogen, amino, lower alkyl or lower alkoxy;

(c) a naphthalene compound of the formula:

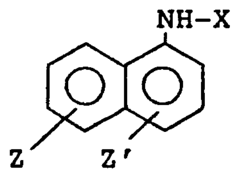

wherein X is hydrogen, methyl or ethyl; and Z and Z′ are independently hydrogen, halogen, amino, lower alkyl or lower alkoxy; and

(d) an anthracene compound of the formula:

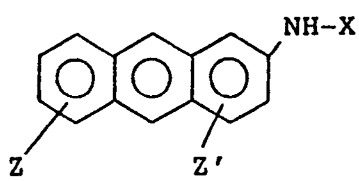

wherein X is hydrogen, methyl or ethyl; and Z and Z′ are independently hydrogen, halogen, amino, lower alkyl or lower alkoxy.

The assay is characterized in that the light output signal, when measured by a luminometer, one minute after the peak light output is attained, is at least about 50% of the intensity of the peak light output (in other words, the t1/2 of light output after peak light output is attained is at least one minute). In preferred methods of the invention, peak light output will be obtained within about 15 minutes, and most preferably, within about 5 minutes of the initiation of the reaction.

The assay kit provided by the invention is for detecting a component of a chemiluminescent reaction involving a chemiluminescent compound, an inorganic peroxide source and an oxidation catalyst as reactants,

the component being one of the chemiluminescent compound, the inorganic peroxide source and the oxidation catalyst. The assay kit comprises a) the reactants other than the component to be assayed; and

b) an amine of the type discussed above;

the amine being further characterized by the fact that it provides a chemiluminescent light output signal which, one minute after peak light output is attained, is at least about 50% of the intensity of said peak light output when the chemiluminescent reaction is conducted in a reaction mixture obtained by combining i) 100 microliters of an aqueous solution containing luminol at a concentration of 0.01 mg/ml and 0.05 M sodium borate/0.01 M EDTA buffer to maintain the pH at 9.5;

ii) 10 microliters of a 0.04 mg/ml aqueous solution of horseradish peroxidase;

iii) 100 microliters of a 0.035 M solution of hydrogen peroxide in 0.001 M EDTA;

iv) 100 microliters of water; and

v) 10 microliters of one and/or the other of a 0.1 mg/ml or a 1 mg/ml aqueous solution of the amine.

The novel assay of the present invention is an extremely sensitive and rapid assay for ligands such as haptens, steroids, antigens, antibodies, proteins and protein containing molecules. The enhanced reactions of the present invention can also be used to detect DNA and small molecules or low molecular weight hormones. When the reactions are carried out in the presence of luminol, they can be used to detect peroxides. It was surprising and very useful to discover excellent utility in amines for not only enhancing the light output of chemiluminescent reactions, but providing sustained enhanced light output. As the result of the sustained enhanced light output provided by the method of the invention, the method provides a very useful and convenient analytical tool.

BRIEF DESCRIPTION OF THE DRAWING

Understanding of the invention will be enhanced by referring to the accompanying drawings in which:

Figure 1 is a plot of the light emission kinetics of a chemiluminescent reaction in accordance with the invention;

Figure 2 is a plot of the background light emission obtained in the presence of an amine;

Figure 3 is a plot of the integrated light output of a chemiluminescent reaction of the invention over a pH range of 7.0 to 10.5;

Figure 4 is a plot of the integrated light output of a chemiluminescent reaction of the invention with varying amounts of an amine added; and

Figure 5 is a plot of light output versus the concentration of an oxidation catalyst using a chemiluminescent reaction of the invention.

DETAILED DESCRIPTION

The present invention describes novel reactions which exhibit chemiluminescent behavior and the utilization of the novel reactions and compositions as analytical assay tools.

Chemiluminescent reactions such as the luminol reaction generally require the presence of a chemiluminescent compound (e.g., luminol in the luminol reaction), an oxidizing agent such as a peroxide, and an oxidization catalyst such as a peroxidase.

As used in the instant specification and claims, the phrase "chemiluminescent compound" designates a compound which accepts energy and emits it in the form of fluorescence in the presence of a peroxide (i.e., as the result of peroxide reaction with the chemiluminescent compound). Chemiluminescent compounds which may be used in the practice of the present invention include luminol and isoluminol; and luminol and isoluminol analogs such as 7-dimethylaminonaphthalene-1,2-dicarboxylic acid hydrazide and those described in U. S. Patent Nos. 4,355,165 and 4,226,993, both patents being incorporated herein by reference.

As far as the inorganic peroxide source is concerned, suitable inorganic peroxides are hydrogen peroxide and sodium perborate.

Suitable oxidation catalysts for use in the present invention include peroxidases such as horseradish peroxidase.

The amines which may be used in the practice of the invention are selected from the group consisting of:

(a) a mono-cyclic aniline compound of the formula:

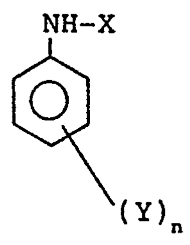

wherein X is hydrogen, methyl, or ethyl; each Y independently is an electron-donating group; and n is zero, one, two or three;

(b) a biphenyl compound of the formula:

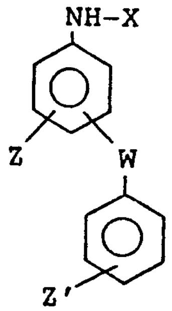

wherein X is hydrogen, methyl or ethyl; W is a carbon-carbon bond, oxygen, methylene, or $-CH_2O-$ and Z and Z′ are independently hydrogen, halogen, amino, lower alkyl or lower alkoxy;

(c) a naphthalene compound of the formula:

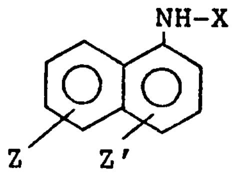

wherein X is hydrogen, methyl or ethyl; and Z and Z′ are independently hydrogen, halogen, amino, lower alkyl or lower alkoxy; and

(d) an anthracene compound of the formula:

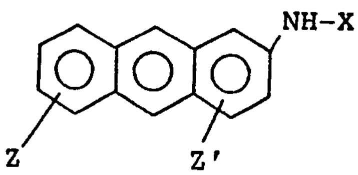

wherein X is hydrogen, methyl or ethyl; and Z and Z′ are independently hydrogen, halogen, amino, lower alkyl or lower alkoxy.

By "electron-donating group", in the context of the Y substituent of the mono-cyclic aniline compound of category (a), is meant a substituent which releases electrons to the aromatic ring through inductive and/or resonance effects.

X is preferably hydrogen or methyl, and is most preferably hydrogen in the amine formulas of categories (a) - (d).

By "lower alkyl" and "lower alkoxy" is meant that the hydrocarbon chain contains one to four carbon atoms. Preferred lower alkyl and lower alkoxy groups are methyl and t-butyl, and methoxy and ethoxy, respectively.

It has been discovered that not only does the enhanced reaction of the present invention produce a brighter light than the normal luminol reaction without an amine, and that the reaction kinetics are such as to provide the emission of light over a longer time period in the form of a sustained enhanced light output, but also that the background light emission, that is, the light produced with no peroxidase present, is substantially reduced by the amines described herein.

The assay is characterized in that the light output signal, when measured by a luminometer, one minute after the peak light output is attained, is at least about 50% of the intensity of the peak light output (in other words, the t1/2 of light output after peak light output is attained is at least one minute). In preferred methods of the invention, peak light output will be obtained within about 15 minutes, and most preferably, within about 5 minutes of the initiation of the reaction.

In an alternative manner, the assay is characterized in that the "enhancement factor" associated with the amine is greater than one, i.e.. the ratio of the light output of an assay with the amine (at a concentration of 0.1 and/or 1.0 mM) to the light output of the assay without the amine is greater than one.

Preferred amines exhibit both a t1/2 of light output (after peak light output is attained) of at least one minute, as well as an enhancement factor of greater than one.

The high intensity, glow reaction characteristic of the instant methods will make chemiluminescent detection of immunoassays simpler and faster. The term "glow reaction" means that the reaction not only produces high intensity light emission, i.e., sufficiently high intensity to be observed often with the naked eye, but also emits at this high intensity for a measurable period of seconds or even minutes rather than only less than five seconds. Since the light output of the glow reaction is usually stable for minutes, activating reagents can be added on the bench top before light measurements are made using a simpler instrument than a conventional luminometer to determine the level of light being emitted. This detection technique is faster than

conventional chemiluminescent, colorimetric or fluorometric assays, and more sensitive than the latter two methods. The dynamic range of these signals is generally two to three orders of magnitude greater than the luminol reaction itself, which may increase the sensitivity of these systems over conventional chemiluminescent immunoassays.

In addition, detection of the chemiluminescent reaction with photographic film is made possible by employment of amines. Exposure of instant films or X-ray films to the light produced by these enhanced reactions for a few minutes is sufficient to produce enough image density for qualitative to semi-quantitative results when visually observed. Quantitative results can also be obtained from these images by use of a low cost densitometer.

It is to be understood, of course, that the methods of the invention themselves may employ any suitable means for determining light output signal, that the light output signal can be analyzed by any suitable method such as by measuring peak height, integrating the area beneath the entire signal or a portion thereof, etc.

The light emission of the chemiluminescent reaction of the invention will be affected to some extent by ancillary factors such as pH, reagent concentration, temperature, method of light measurement and the like. In order to obtain optimum results, these factors should be controlled and regulated to the extent possible.

At the present time, it is believed that the chemiluminescent reaction of the invention is not as dependent on variations of pH as other similar assay reactions. Generally, it has been found that pH's from about 7.0 to about 13.0 are suitable. It is presently preferred that the pH be about 8 to 10.5, and most preferred is a pH of 8.5 to 9.0. Suitable buffering agents for use in maintaining a selected pH are borate, carbonate, phosphate, tris(hydroxymethyl)aminomethane and the like.

Generally speaking, the range of reagent concentrations for use in the assay reactions are:
Oxidation catalyst 0.01 micrograms to 5000 mg/l
Peroxide source 10 micromole to 300 millimole/l
Chemiluminescent compound 0.5 micromole to 200 millimole/l
Amine 10 micromole to 100 millimole/l

The intensity of the light output and duration of the glow reaction will depend to some extent upon the particular components of the chemiluminescent reaction chosen and the amounts of each employed. In particular, the nature of the light output seems to be influenced significantly by the amount of the amine employed.

The temperature under which the assay is carried out to achieve optimum results should be moderate, for example, from about 0 to 50°C, and preferably about 20°C.

The enhanced chemiluminescent reaction described herein is also very useful in analytical assays for components of the reaction such as a chemiluminescent compound, a peroxide source or an oxidation catalyst.

The enhanced chemiluminescent reaction described herein is also useful in immunoassays wherein the label is a chemiluminescent compound (e.g., luminol) or a catalyst for the chemiluminescent reaction. These reactions are made more sensitive, that is, the component being assayed for is more readily detected and quantitated due to the greater light output resulting from the presence of the amine.

Examples of known luminol-based immunoassays are assays for steroids such as progesterone; for biotin and avidin; for thyroxine; and for hepatitis B surface antigen. These assays are described in the book "Luminescent Assays: Perspectives in Endocrinology and Clinical Chemistry" edited by M. Serio and M. Pozzagli, Raven Press, New York, 1982.

An example of luminol-reaction-based assays wherein the labeled species is horseradish peroxidase, include protein binding assays, is described by Olsson et al., J. Immunological Methods, 25, 127-135 (1979).

The usefulness of the enhancement of chemiluminescent reactions as a method for improving assays can be explained more fully as follows: A chemiluminescent reaction requires or utilizes several substrates. One or more (generally all) of the substrates may be critical or rate-limiting. The substrates may be bound or unbound, i.e., in some cases, substrates may be bound to other substances, for example, drugs, antibodies, proteins, antigens, DNA and the like, in order to provide a label by which such other substances may be detected.

In the example of the luminol reaction, free luminol or luminol bound to some other substance may be detected. This reaction would be enhanced by the method of the invention.

Because a peroxide source is a necessary component in the chemiluminescent reaction, the presence of the peroxide source may be shown by the occurrence of the chemiluminescent reaction. Thus, the method of the invention will enhance, for example, the detection of hydrogen peroxide or substrates that generate hydrogen peroxide.

Further, all of the reactions of U. S. Patent No. 4,220,450 which involve a chemiluminescent reaction would offer opportunities for the use of the present invention, that is, the ligand or anti-ligand described in U. S. Patent No. 4,220,450 could be labelled with a component of the chemiluminescent reaction, and the ligand or anti-ligand detected more readily as a result of enhancement of the chemiluminescent reaction using an amine.

The assay kits of the present invention for detecting a component of a chemiluminescent reaction comprise i) the reactants of the chemiluminescent reaction other than the component to be assayed for; and ii) an amine. Such reactants and amines are usually present in sufficient quantities to conduct a plurality of assays, and are packaged in substantially stable form (i.e., the kit exhibits a shelf-life of at least about 30 days at room temperature).

The assay kit will also generally contain one or more of the preferred buffer solutions used to adjust the pH of the analytical reaction mixture. The kit may optionally contain one or more standard solutions of known amounts of substances to be assayed, or standard curves showing assays run under standard conditions. The kit may also include a container suitable for carrying out the assay reaction in the apparatus used to measure the light output.

The resulting assays may be useful in both the doctor's office and the clinical laboratory. Conventional chemiluminescent reactions, e.g., those using luminol, oxalate, or acridinium derivatives, have kinetics which produce intense light spikes which rapidly decay away or produce low level longer emissions. Both types of kinetics require special instrumentation, i.e., the activating reagents need to be injected into each sample in the measuring chamber followed by an observation period to integrate or find the peak light level. This can take anywhere from 5 to 20 seconds per sample, and no multiple sample instruments are commercially available to speed the process. Assays using amines, due to the glow reaction produced, can be activated on the bench top and then placed in a simple instrument, such as a Lumac® Biocounter 2010 or Lumac® Biocounter M3000 (luminometers available from 3M, St. Paul, Minnesota), to read the light levels of a sample in seconds. The speed of the technique can be increased for clinical laboratory use where a microtiter plate (96 determinations) can be read in under a minute by a microtiter plate reader.

All of the luminometer measurements in the Examples were carried out on a Lumac® Biocounter M3000.

The following Examples are offered by way of illustration and not by way of limitation.

EXAMPLE I

A mixture of 100 microliters of luminol (0.01 mg/ml in 0.05 M borate buffer with 0.01 M ethylenediamine tetraacetic acid (EDTA) pH 9.5), 10 microliters of an amine (either 0.1 or 1.0 mg/ml in water), 100 microliters of water and 10 microliters of water or horseradish peroxidase (HRP) (0.04 mg/ml) was placed in a luminometer tube and 100 microliters of hydrogen peroxide (0.02 M in 0.001 M EDTA) was added in the detection chamber of a luminometer. The light output of the reaction using p-anisidine was monitored for three minutes and plotted versus time in FIGURE 1, the dotted line representing the light output observed in the presence of p-anisidine and the solid line representing the blank. Signal to background (S/B) ratios were determined by comparing the light produced with and without HRP. These data, in TABLE 1, show that the addition of the indicated amines changes the luminol reaction's light production dramatically with higher intensity emission over a much longer period of time (as evidenced by the t1/2 after peak light output is attained). FIGURE 2 further shows that p-anisidine actually reduces background light output as the dotted line shows greatly reduced background light of the luminol reaction with p-anisidine and the solid line shows the background light of the luminol reaction without p-anisidine. In both reactions, no horseradish peroxidase is present. The light output units are relative light units as measured on a Lumac® Biocounter. The absence of a value in TABLE 1 indicates that a particular measurement was not made.

TABLE 1

SUMMARY OF LIGHT OUTPUTS OF LUMINOL REACTION WITH ANILINE-BASED COMPOUNDS

| Amine Added | Integrated Light Units With HRP | Integrated Light Units With No HRP | t1/2 (seconds) | S/B Ratio | Enhancement Factor |
|---|---|---|---|---|---|
| None (Blank) | 60,787.2 | 414.4 | 6 | 146.7 | |
| Aniline | 567,785.4 | 22.8 | 96 | 24,888.3 | 9.3 |
| N-Methylaniline | 177,444.1 | 7.5 | >180 | 23,523.3 | 2.9 |
| o-Toluidine | 599,096.6 | 24.2 | 116 | 24,803.9 | 9.9 |
| m-Toluidine | 234,565.0 | -- | 60 | -- | 22.9 |
| p-Toluidine | 628,221.8 | -- | >180 | -- | 118.4 |
| p-(t-Butyl)aniline | 527,656.7 | 17.7 | 120 | 29,749.5 | 8.7 |
| o-Anisidine | 954,133.2 | 46.7 | >180 | 20,454.5 | 15.7 |
| p-Anisidine | 909,857.2 | 12.4 | >180 | 73,080.9 | 15.0 |
| p-Phenetidine | 3,204.2 | -- | >180 | -- | 2.8 |
| N-Methyl-p-anisidine | 64,899.7 | -- | >180 | -- | 22.7 |
| o-Phenylenediamine | 377,661.3 | 2.1 | >180 | 183,034.5 | 6.2 |
| 2,4-Dimethoxyaniline | 4,361.7 | -- | >180 | -- | 3.4 |
| 3,4-Dimethoxyaniline | 51,418.9 | -- | >180 | -- | 7.4 |
| 4-Methoxy-2-methylaniline | 74,344.9 | -- | >180 | -- | 11.7 |
| 3,4-Diaminotoluene | 44,658.3 | 3.6 | >180 | 12,291.3 | .73 |
| 3,4-Methylenedioxyaniline | 30,734.0 | -- | >180 | -- | 5.5 |
| 5-Phenyl-o-anisidine | 647,460.0 | 8.9 | 36 | 72,503.9 | 10.6 |
| o-Aminobiphenyl | 559,662.7 | 19.3 | 40 | 28,983.1 | 9.2 |
| p-Aminobiphenyl | 823,988.7 | 63.3 | 58 | 12,814.8 | 13.6 |

**TABLE 1** (cont.)

**SUMMARY OF LIGHT OUTPUTS OF LUMINOL REACTION WITH ANILINE-BASED COMPOUNDS**

| Amine Added | Integrated Light Units With HRP | Integrated Light Units With No HRP | t1/2 (seconds) | S/B Ratio | Enhancement Factor |
|---|---|---|---|---|---|
| 4-Aminophenyl ether | 35,587.5 | -- | 74 | -- | 11.7 |
| 4-Benzyloxyaniline | 177,738.7 | -- | 146 | -- | 32.0 |
| Benzidine | 211,795.5 | 2.1 | 150 | 99,746.7 | 3.5 |
| 3,3′-Diaminobenzidine | 859,459.2 | 15.9 | >180 | 54,031.4 | 14.1 |
| 4,4′-Methylenedianiline | 879,798.8 | 255.5 | >180 | 3,442.8 | 14.5 |
| 3,3′,5,5′-Tetramethyl-benzidine | 812,216.3 | -- | >180 | -- | 78.8 |
| 1-Aminonaphthalene | 927,584.0 | 14.3 | >180 | 64,655.0 | 15.3 |
| 1,5-Diaminonaphthalene | 643,602.7 | 10.8 | >180 | 59,629.6 | 4.3 |
| 1-Amino-4-chloronaphthalene | 900,428.7 | 44.0 | >180 | 20,444.2 | 6.1 |
| 2 Aminoanthracene | 312,935.8 | | >180 | | 63.4 |

EXAMPLE 2

The procedures of EXAMPLE 1 were repeated using the amines indicated in TABLE 2 below at a concentration of 0.1 mg/ml. As noted in TABLE 2, those amines all provided a t1/2 of less than 60 seconds (after peak light output was attained). Though not included as data in TABLE 2, increasing the concentration of amines indicated in TABLE 2 to 1 mg/ml did not significantly alter the t1/2 (i.e., the t1/2 of at least 60 seconds, as required in the instant invention, was not obtained).

**TABLE 2**

| Compound Added (0.1 mg/ml) | Integrated Light Units with HRP | Integrated Light Units without HRP | t1/2 Seconds | S/B Ratio |
|---|---|---|---|---|
| None | 60,787.2 | 414.4 | 6 | 146.7 |
| o-Nitroaniline | 57,584.0 | 125.8 | 6 | 191.7 |
| m-Nitroaniline | 48,134.4 | 255.5 | 6 | 188.4 |
| p-Nitroaniline | 61,181.3 | 285.3 | 6 | 214.0 |
| m-Anisidine | 104,638.1 | 54.2 | 16 | 1,930.9 |
| o-Chloroaniline | 49,431.0 | 213.4 | 6 | 231.6 |
| o-Bromoaniline | 40,447.5 | 162.5 | 6 | 249.0 |
| o-Iodoaniline | 29,886.3 | 149.5 | 4 | 200.0 |
| m-Phenylenediamine | 168,458.7 | 61.4 | 10 | 2,744.2 |
| p-Phenylenediamine | 6,609.8 | 2.8 | 4 | 2,357.8 |
| 2,4-Diaminophenol | 8,906.3 | 15.7 | 8 | 566.9 |
| o-Aminophenol | 974.3 | 0.9 | 8 | 1,036.5 |

EXAMPLE 3

Enhanced luminol reactions were carried out over the pH range of 7.0 to 10.5 in 0.1 M tris(hydroxymethyl)aminomethane buffer containing 0.01 milligrams per milliliter of luminol and 0.01 M ethylenediaminetetraacetic acid. The reaction mixture contained 10 microliters of horseradish peroxidase (0.04 milligrams per milliliter), 10 microliters of p-anisidine (0.1 milligrams per milliliter), 100 microliters of water and 100 microliters of luminol solution. In the luminometer, 100 microliters of hydrogen peroxide, 0.035 M in 0.001 M ethylenediaminetetraacetic acid, was injected into the sample and the light produced by the reaction was monitored for 3 minutes. The luminol reaction without added p-anisidine was also run in a similar manner with the same solutions with the exception that 10 microliters of water was substituted for the anisidine. The integrated light outputs of these reactions were then plotted versus the reaction pH in FIGURE 3 and show that the more useful light outputs were observed above pH 8.0 with p-anisidine (dotted line) while the unenhanced luminol reaction produced minimal light from pH 7 to 9.5 (solid line). A similar result was shown in 0.1 M sodium bicarbonate buffer. The peak light production occurred at about pH 8.5-9.0 for these p-anisidine-enhanced reactions.

EXAMPLE 4

The concentration of p-anisidine was varied from 0.5 to 0.01 milligrams per milliliter (4.10 to 0.081 millimolar) when added to a luminol reaction consisting of 100 microliters of 0.01 milligrams per milliliter of luminol in 0.1 M tris(hydroxymethyl)aminomethane and 0.01 M ethylenediaminetetraacetic acid at pH 9.0, 10 microliters of horseradish peroxidase, 0.02 milligrams per milliliter in water, 100 microliters of water, and 10 microliters of p-anisidine. The reaction was started by injection of 100 microliters of hydrogen peroxide, 0.035 M in 0.001 M ethylenediaminetetraacetic acid, and the light produced by the reaction monitored for 3 minutes. FIGURE 4 shows the integrated light outputs for these reactions plotted versus the concentration of p-anisidine added to the reaction mixture. It shows that the enhanced light output effect is greatest at about 0.00041 M or 0.05 milligrams per milliliter of p-anisidine.

EXAMPLE 5

A titration of horseradish peroxidase was run by adding 20 microliter aliquots of solutions containing from 7.0 micrograms to 1.7 nanograms per milliliter of horseradish peroxidase in water, to a mixture of 500 microliters of 0.01 milligrams per milliliter of luminol in 0.1 M sodium bicarbonate and 0.01 M ethylenediaminetetraacetic acid at pH 8.5 and 10 microliters of 0.00041 M p-anisidine in water. The reaction was started by injection of 100 microliters of hydrogen peroxide, 0.035 M in 0.001 M ethylenediaminetetraacetic acid, in a luminometer and monitored for 3 minutes. Samples were run in triplicate, the light output curves averaged, and the net light levels (sample light level minus the light level without added peroxidase) calculated. FIGURE 5 shows a plot of horseradish peroxidase concentration versus the average net light level at 30 seconds (solid line) and 90 seconds (dotted line) after injection of hydrogen peroxide. This plot and those for the light level at 60, 120, 150 and 180 seconds after injection which are very similar with nearly the same slopes and intercepts show that the concentration of peroxidase is related to the light produced by the chemiluminescent reaction of the invention at nearly any time during the reaction. These results indicate that the addition of p-anisidine to the luminol reaction produces enhanced chemiluminescence which is analytically useful and can be measured at many time points to produce an analytical result.

## Claims

1. A method for enhancing the sensitivity of an assay reaction for the detection of a component of a chemiluminescent reaction involving a chemiluminescent compound, an inorganic peroxide source and an oxidation catalyst, said component being one of said chemiluminescent compound, said peroxide source, and said oxidation catalyst, said method comprising including an amine in said assay reaction, said amine selected from the group consisting of:

(a) a mono-cyclic aniline compound of the

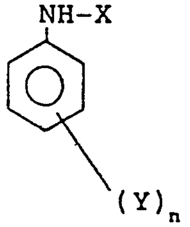

wherein X is hydrogen, methyl, or ethyl; each Y independently is an electron-donating group; and n is zero, one, two or three;

(b) a biphenyl compound of the formula:

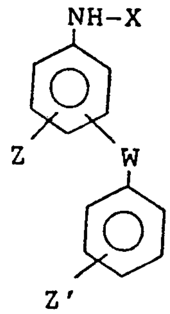

wherein X is hydrogen, methyl or ethyl; W is a carbon-carbon bond, oxygen, methylene, or $-CH_2O-$; and Z and Z' are independenetly hydrogen, halogen, amino, lower alkyl or lower alkoxy;

(c) a naphthalene compound of the formula:

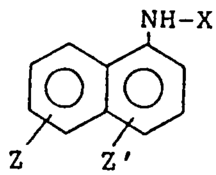

wherein X is hydrogen, methyl or ethyl; and Z and Z' are independently hydrogen, halogen, amino, lower alkyl or lower alkoxy; and

(d) an anthracene compound of the formula:

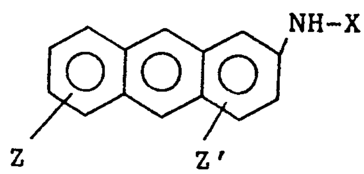

wherein X is hydrogen, methyl or ethyl; and Z and Z' are independently hydrogen, halogen, amino, lower alkyl or lower alkoxy, said amine being further characterized by the fact that it provides a light output signal which, one minute after peak light output is attained, is at least about 50% of the intensity of said peak light output.

2. A method according to claim 1, wherein said oxidation catalyst is a peroxidase.

3. A method according to claim 2, wherein said oxidation catalyst is horseradish peroxidase.

4. A method according to claim 1, wherein said inorganic peroxide source is hydrogen peroxide.

5. A method according to any one of claims 1 to 4, wherein said chemiluminescent compound is selected from the group consisting of luminol, a luminol analog, isoluminol and an isoluminol analog.

6. An assay kit for detecting a component of a chemiluminescent reaction involving a chemiluminescent compound, an inorganic peroxide source and an oxidation catalyst as reactants, said component being one of said chemiluminescent compound, said inorganic peroxide source and said oxidation catalyst, said assay kit comprising:

a) said reactants other than said component; and

b) an amine selected from the group consisting of:

(a) a mono-cyclic aniline compound of the formula:

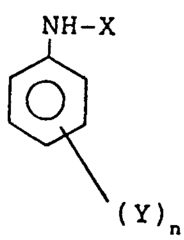

wherein X is hydrogen, methyl, or ethyl; each Y independently is an electron-donating group; and n is zero, one, two or three;

(b) a biphenyl compound of the formula:

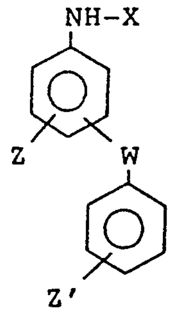

wherein X is hydrogen, methyl or ethyl; W is a carbon-carbon bond, oxygen, methylene, or $-CH_2O-$; and Z and Z′ are independently hydrogen, halogen, amino, lower alkyl or lower alkoxy;

(c) a naphthalene compound of the formula:

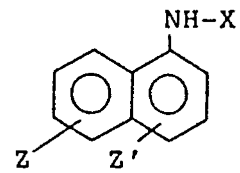

wherein X is hydrogen, methyl or ethyl; and Z and Z′ are independently hydrogen, halogen, amino, lower alkyl or lower alkoxy; and

(d) an anthracene compound of the formula:

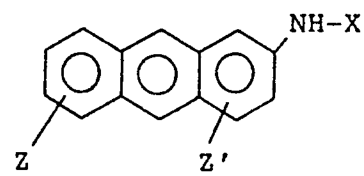

wherein X is hydrogen, methyl or ethyl; and Z and Z′ are independently hydrogen, halogen, amino, lower alkyl or lower alkoxy; said amine being further characterized by the fact that it provides a chemiluminescent light output signal which, one minute after peak light output is attained, is at least about 50% of the intensity of said peak light output when the chemiluminescent reaction is conducted in a reaction mixture obtained by combining

i) 100 microliters of an aqueous solution containing luminol at a concentration of 0.01 mg/ml and 0.05 M sodium borate/0.01 M EDTA buffer to maintain the pH at 9.5;

ii) 10 microliters of a 0.04 mg/ml aqueous solution of horseradish peroxidase;

iii) 100 microliters of a 0.035 M solution of hydrogen peroxide in 0.001 M EDTA;

iv) 100 microliters of water; and

v) 10 microliters of one and/or the other of a 0.1 mg/ml or a 1 mg/ml aqueous solution of the amine.

7. A method according to any one of claims 1 to 6 wherein the oxidation catalyst or the chemiluminescent compound is coupled to a ligand.

8. A method according to any one of claims 1 to 7 wherein the assay is an immunoassay.

9. A method according to any one of claims 1 to 8 wherein the ligand is an antibody.

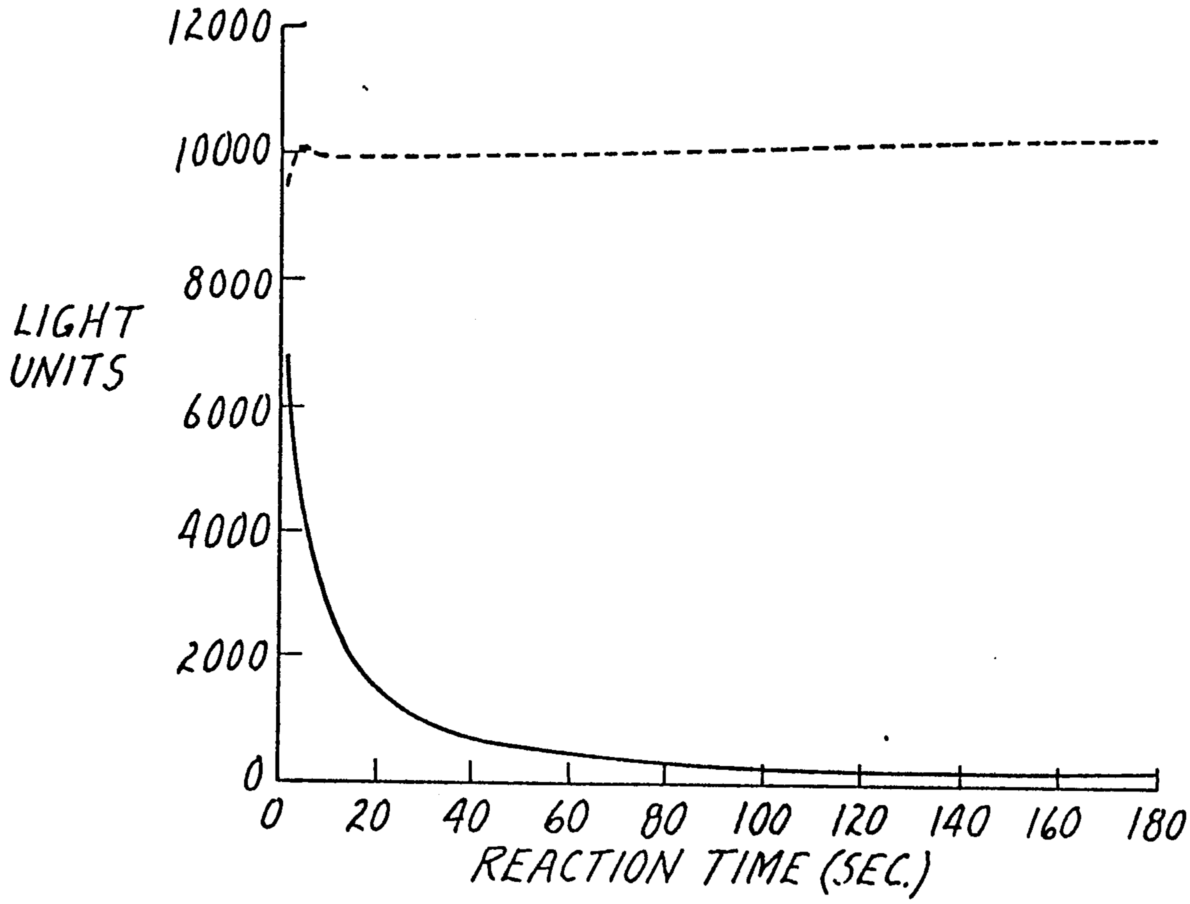
12000
10000
8000
LIGHT UNITS
6000
4000
2000
0
0 20 40 60 80 100 120 140 160 180
REACTION TIME (SEC.)

FIG. 1

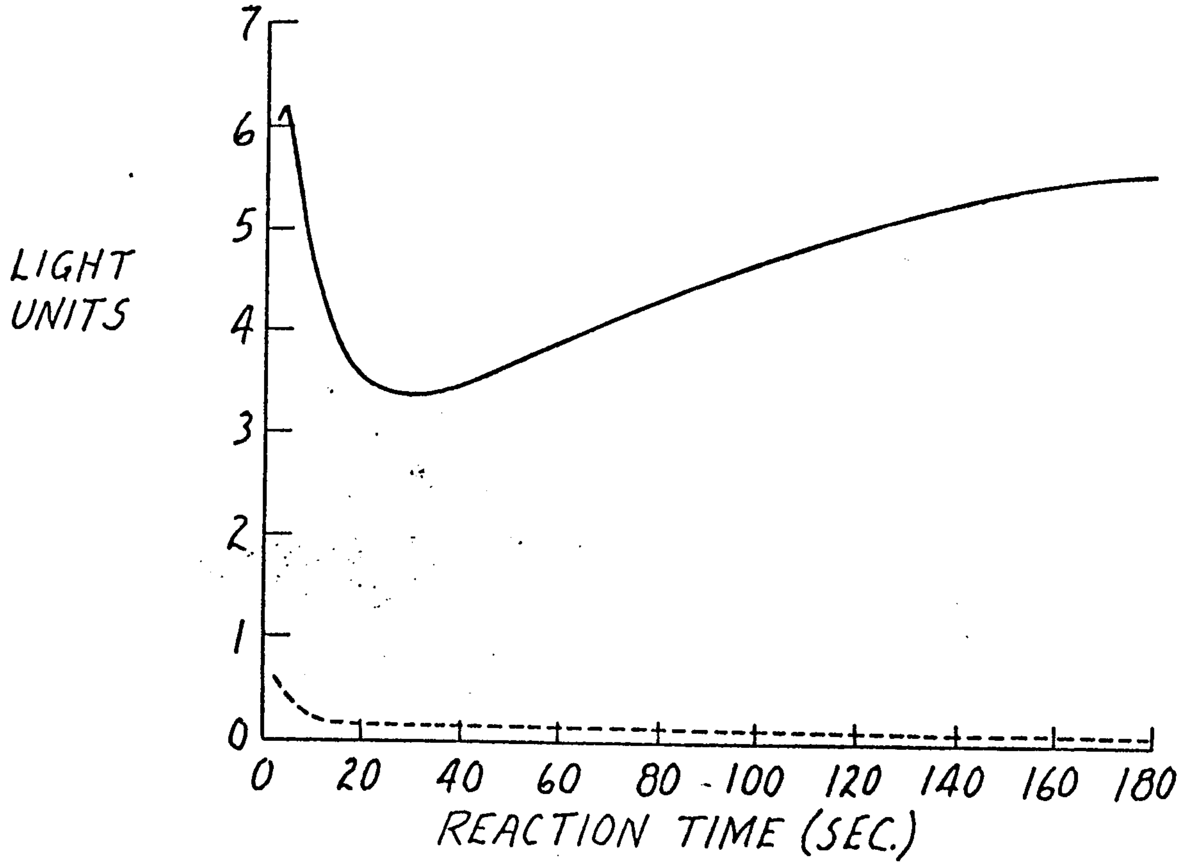
7
6
5
LIGHT UNITS
4
3
2
1
0
0 20 40 60 80 100 120 140 160 180
REACTION TIME (SEC.)

FIG. 2

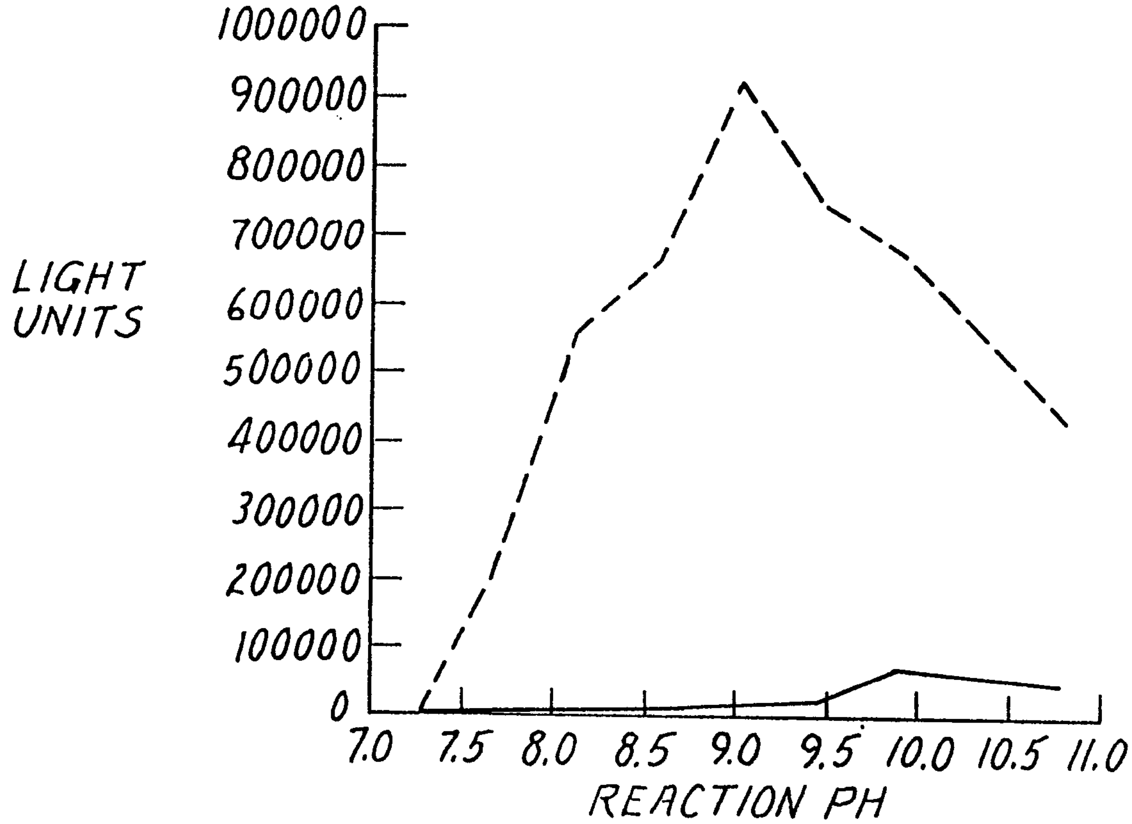
LIGHT UNITS
1000000
900000
800000
700000
600000
500000
400000
300000
200000
100000
0
7.0 7.5 8.0 8.5 9.0 9.5 10.0 10.5 11.0
REACTION PH

FIG. 3

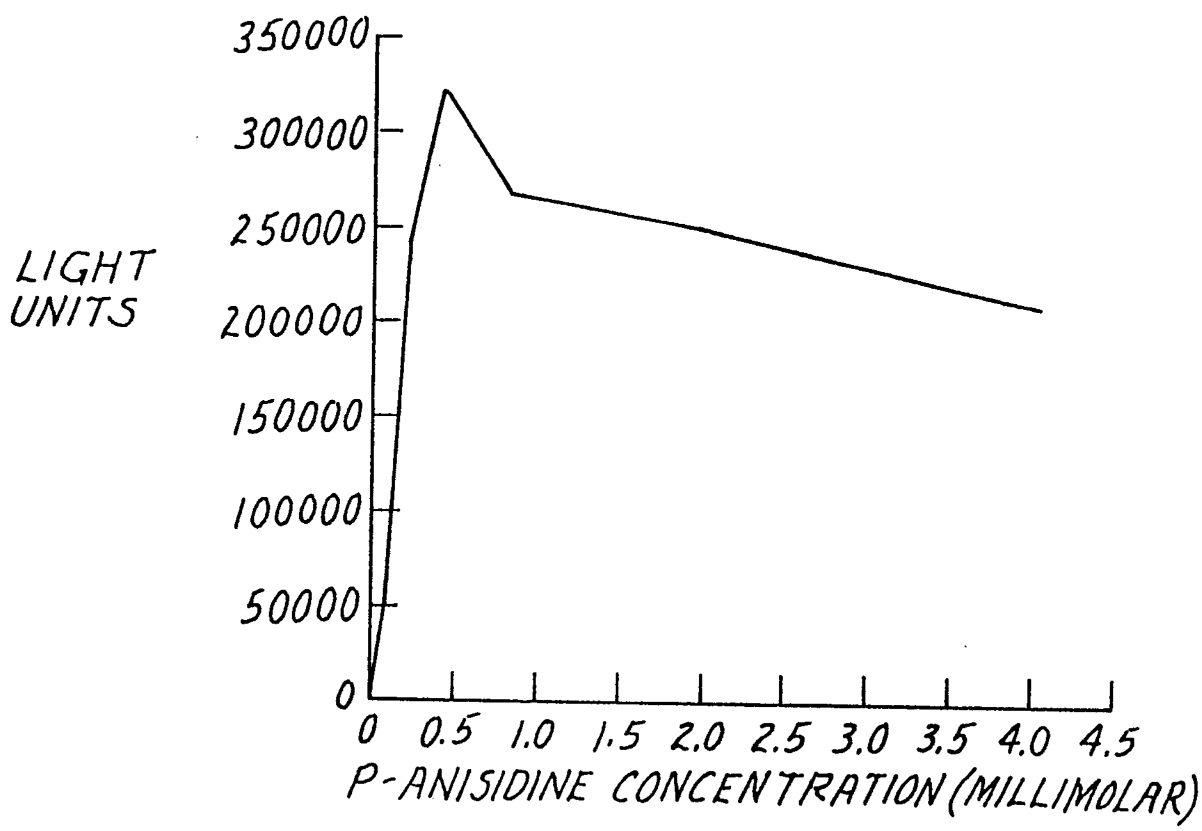
LIGHT UNITS
350000
300000
250000
200000
150000
100000
50000
0
0 0.5 1.0 1.5 2.0 2.5 3.0 3.5 4.0 4.5
P-ANISIDINE CONCENTRATION (MILLIMOLAR)

FIG. 4

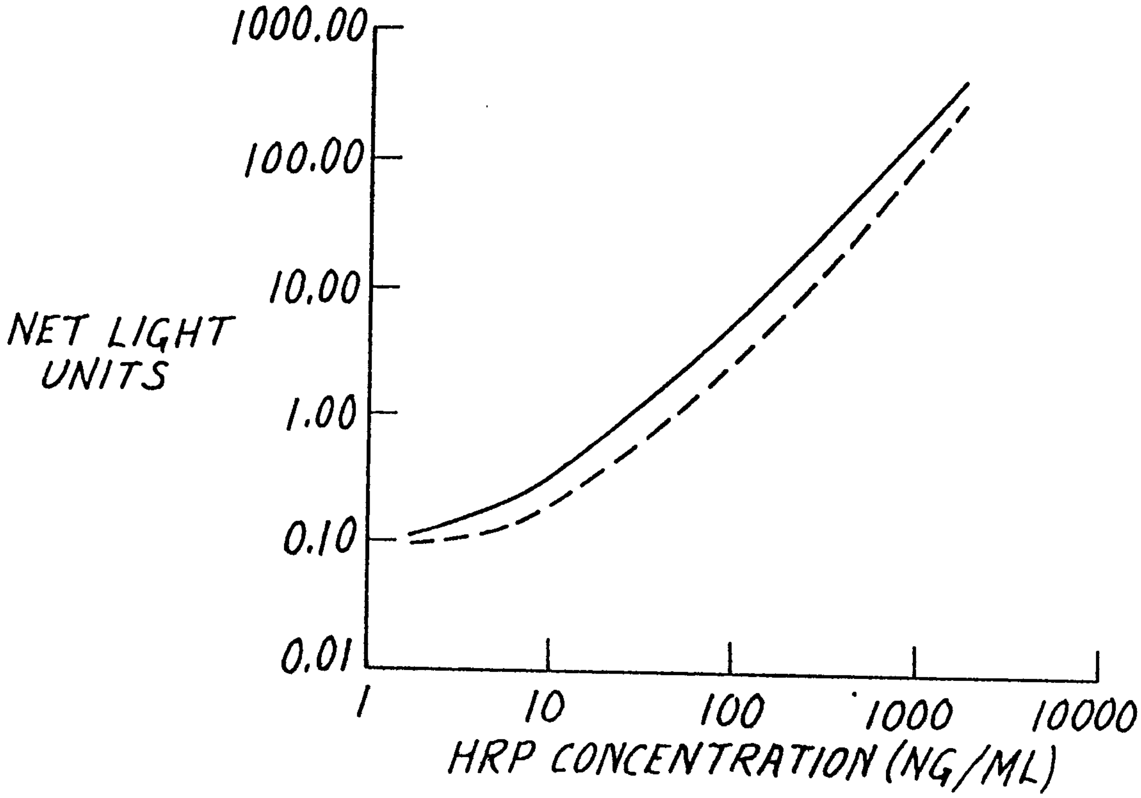
1000.00
100.00
10.00
NET LIGHT UNITS
1.00
0.10
0.01
1
10
100
1000
10000
HRP CONCENTRATION (NG/ML)

FIG. 5